# EUROPÄISCHE PATENTSCHRIFT

(11) **EP 4 212 451 B1**
(45) Veröffentlichungstag und Bekanntmachung des Hinweises auf die Patenterteilung: **11.09.2024**
(21) Anmeldenummer: 23158837.7
(22) Anmeldetag: 02.10.2020
(51) Int. Cl.: B65D 47/18, B65D 47/20, B05B 11/04

(54) **TROPFENSPENDER**
DROP DISPENSER
DISTRIBUTEUR DE GOUTTES

(43) Veröffentlichungstag der Anmeldung: 19.07.2023
(62) Teilanmeldung aus: 20199907.5
(73) Patentinhaber: Aptar Radolfzell GmbH, 78315 Radolfzell (DE)
(72) Erfinder: Greiner-Perth, Jürgen, 78244 Gottmadingen (DE)
(74) Vertreter: Witte, Weller und Partner Patentanwälte mbB Stuttgart

(56) Entgegenhaltungen:
- WO-A1-2018/141350
- DE-A1- 102018 124 067
- DE-U1- 202012 012 770
- US-A1- 2011 155 770
- US-B2- 8 863 998

## Beschreibung

### ANWENDUNGSGEBIET UND STAND DER TECHNIK

Die Erfindung betrifft einen Flüssigkeitsspender, insbesondere in Art eines Tropfenspenders. Ein erfindungsgemäßer Flüssigkeitsspender findet insbesondere zur Abgabe pharmazeutischer Flüssigkeiten Verwendung. In Ausgestaltung als Tropfenspender kann ein erfindungsgemäßer Flüssigkeitsspender insbesondere zur ophthalmischen Applikation von pharmazeutischen Flüssigkeiten Verwendung finden.

Ein gattungsgemäßer Flüssigkeitsspender verfügt über einen Flüssigkeitsspeicher und einen hieran befestigten Austragkopf. Der gattungsgemäße Austragkopf umfasst eine Austragöffnung sowie ein der Austragöffnung vorgeschaltetes Auslassventil, welches in Folge von Flüssigkeitsdruck in einer Druckkammer unter Verlagerung eines Ventilkörpers öffnet, so dass Flüssigkeit durch die Austragöffnung hindurch austreten kann. Der Ventilkörper verkleinert beim Öffnen eine der Druckkammer entgegengesetzt angeordnete Ventilkammer. Der Flüssigkeitsspeicher ist bei einem gattungsgemä-ßen Flüssigkeitsspender zum Zwecke des Druckausgleichs mit einer umgebenden Atmosphäre zumindest zeitweise verbunden. Er weist einen Speicherbelüftungskanal auf, um insbesondere das Nachströmen von Luft in den Flüssigkeitsspeicher nach dem Flüssigkeitsaustrag zu gestatten.

Je nach Gestaltung ist das Ventil zum Öffnen bei bereits geringem Überdruck in der Druckkammer ausgebildet. Insbesondere bei Tropfenspendern ist es erforderlich, einen genau dosierten Austrag der Flüssigkeit zu gestatten. Dies wird durch ein bei geringem Überdruck öffnendes Auslassventil begünstigt.

DE 10 2018 124067 A1 offenbart einen Tropfenspender gemäß dem Oberbegriff des Anspruchs 1.

### AUFGABE UND LÖSUNG

Aufgabe der Erfindung ist es, einen gattungsgemäßen Spender dahingehend weiterzubilden, dass das Öffnungsverhalten des bei vergleichsweise geringem Überdruck öffnenden Auslassventils besser abstimmbar ist.

Erfindungsgemäß wird hierfür ein Flüssigkeitsspeicher vorgeschlagen, der in der bereits beschriebenen Art einen Flüssigkeitsspeicher und einen am Flüssigkeitsspeicher befestigten Austragkopf aufweist. Der Flüssigkeitsspeicher wird vorzugsweise durch einen Flaschenkörper aus Kunststoff gebildet, insbesondere in Art einer zusammendrückbaren Quetschflasche (Squeeze Bottle). Der Austragkopf kann einstückig mit dem Flaschenkörper verbunden sein. Vorzugsweise ist er jedoch durch eine Schnapp- oder Gewindeverbindung mit dem Flaschenkörper verbunden.

Der Austragkopf weist die Austragöffnung auf, die über einen Austragkanal mit dem Flüssigkeitsspeicherverbunden ist. Im Austragkanal ist das Auslassventil vorgesehen. Dieses verfügt über einen Ventilkörper, der durch Verformung oder Verlagerung als Ganzes gegenüber der Austragöffnung verlagerbar ist, um diese zu öffnen und zu schließen. Der Ventilkörper ist zwischen der Druckkammer, die Teil des Austragkanals ist, und der Ventilkammer angeordnet. Bei Druckbeaufschlagung wird der Ventilkörper zumindest teilweise in Richtung der Ventilkammer verlagert und verkleinert diese. Entfällt der Überdruck, so gelangt der Ventilkörper wieder in seine die Austragöffnung verschließende Grundposition, vorzugsweise in Folge einer elastischen Rückverformung des Ventilkörpers oder kraftbeaufschlagt durch eine separate Rückstellfeder. Insbesondere kann es sich um eine in der genannten Ventilkammer angeordnete Rückstellfeder handeln.

Der erfindungsgemäße Austragkopf verfügt über zwei Kanäle, die beide dem Druckausgleich dienen. Zum Zwecke des Druckausgleichs im Flüssigkeitsspeicher ist der Speicherbelüftungskanal vorgesehen und zum Zwecke des Druckausgleichs in der Ventilkammer ist ein Ventilbelüftungskanal vorgesehen. Die beiden Kanäle sind getrennt voneinander ausgebildet. Sie weisen demzufolge voneinander getrennte Einlässe auf, welche an einer Außenseite des Austragkopfes vorgesehen sind.

Durch diese vollständige Trennung der beiden Belüftungskanäle wird verhindert, dass die Druckverhältnisse in der Ventilkammer einerseits und im Flüssigkeitsspeicher andererseits ungewollte Auswirkungen haben. So wird beispielsweise verhindert, dass während des Austrags der Überdruck im Flüssigkeitsspeicher die Öffnungsneigung des Auslassventils senkt und/oder dass ein nach dem Austrag noch bestehender Unterdruck im Flüssigkeitsspeicher die Öffnungsneigung des Ventils ungewollt begünstigt.

Der Austragkopf weist vorzugsweise ein Innenbauteil und ein Außenbauteil auf, welches in einer Montagerichtungaufdas Innenbauteil aufgeschoben und dort vorzugsweise verrastet ist. Das Innenbauteil und das Außenbauteil können gemeinsam einen Aufnahmeraum für den Ventilkörper aufweisen, wobei vorzugsweise die Ventilkammer zwischen dem Ventilkörper und dem Innenbauteil und die Druckkammer zwischen dem Ventilkörper und dem Außenbauteil gebildet ist.

DerSpeicherbelüftungskanal und derVentilbelüftungskanal erstrecken sich vorzugsweise durch das Innenbauteil und das Außenbauteil.

Das Außenbauteil ist vorzugsweise von den beiden Einlässen durchdrungen. Das Innenbauteil weist an seiner Außenseite vorzugsweise zwei Öffnungen auf, die mit den Einlässen am Außenbauteil in kommunizierender Verbindung stehen. Dabei kann insbesondere vorgesehen sein, dass einer der beiden Kanäle oder beide Kanäle einen Kanalteilabschnitt aufweisen, der als ringabschnittsförmiger Kanalteilabschnitt zwischen dem Außenbauteil und dem Innenbauteil gebildet ist. Ein solcher Kanalteilabschnitt kann die Montage von Innenbauteil und Außenbauteil erleichtern, da er eine variablere Relativanordnung der Bauteile gestattet.

Die Einlässe der beiden Kanäle an der Außenseite des Außenbauteils können relativ zueinander verschieden ausgerichtet sein. Sie können bezogen auf den Umfang eine übereinstimmende oder benachbarte Position aufweisen, also auf der gleichen Seite des Außenbauteils vorgesehen sein. Dies erleichtert die Herstellung des Außenbauteils als Spritzgussteil, da für beide Einlässe nur ein gemeinsamer Schieber am Werkzeug benötigt wird.

Vorzugsweise sind die beiden Einlässe in Montagerichtung der Bauteile versetzt zueinander angeordnet. Eine solche versetzte Anordnung ermöglicht eine ebenfalls in Montagerichtung versetzte Anordnung der jeweiligen Kanalabschnitte im Innenbauteils.

Die Kanalabschnitte des Speicherbelüftungskanals und des Ventilbelüftungskanals im Innenbauteil weisen vorzugsweise jeweils geradlinige Kanalteilabschnitte auf, die das Innenbauteil durchdringen und an seiner Außenseite münden. Bezogen auf eine Ebene, deren Normalenvektor mit einer Austragrichtung und/oder der Montagerichtung übereinstimmt, sind diese geradlinigen Kanalteilabschnitte vorzugsweise gegeneinander angewinkelt oder insbesondere parallel und beabstandet zueinander angeordnet, um sie getrennt voneinander bei geringem verfügbarem Raum im Innenbauteil vorsehen zu können.

Vorzugsweise kann vorgesehen sein, dass zwischen den Einlässen an der Außenseite des Austragkopfes eine umlaufende Stufe vorgesehen ist. Diese Stufe kann auf eine Schutzkappe des Flüssigkeitsspeichers derart abgestimmt sein, dass die Innenseite der Schutzkappe bei aufgesetzter Schutzkappe dichtend an der Stufe anliegt und somit die Einlässe voneinander trennt.

Eine abnehmbare Schutzkappe, die Teil des Flüssigkeitsspenders ist und im aufgesetzten Zustand die Austragöffnung schützt, ist demnach vorzugsweise derart ausgebildet, dass sie im aufgesetzten Zustand durch Anlage am Außenbauteil und insbesondere an der genannten Stufe die beiden Einlässe voneinander isoliert. Insbesondere kann sie derart ausgebildet sein, dass sie im aufgesetzten Zustand mindestens einen der Einlässe und vorzugsweise beide Einlässe getrennt voneinander gegenüber einer umgebenden Atmosphäre isoliert. Durch die Isolation der Einlässe voneinander bei aufgesetzter Schutzkappe wird verhindert, dass sich der Druck im Flüssigkeitsspeicher und in der Ventilkammer bei aufgesetzter Schutzkappe gegenseitig beeinflussen.

Wie bereits erläutert, kann der Flüssigkeitsspender insbesondere als Tropfenspender ausgebildet sein und vorzugsweise mit einer pharmazeutischen Flüssigkeit zur ophthalmischen Applikation befüllt sein.

In der Ausgestaltung als Tropfenspender weistder Flüssigkeitsspender vorzugsweise ein Tropfenbildungsmittel im Bereich der Austragöffnung auf. Hierbei kann es sich um eine trichterförmige und sich stromabwärts öffnende Struktur handeln. Vorzugsweise jedoch umfasst das Tropfenbildungsmittel eine die Austragöffnung außenseitig umgebende Tropfenbildungsfläche, die zumindest im Wesentlichen flach ausgebildet ist. Außenseitig ist die Tropfenbildungsfläche vorzugsweise durch eine Abrisskante umgeben.

Bei einem erfindungsgemäßen Flüssigkeitsspender, insbesondere in der Ausgestaltung als Tropfenspender, kann der Ventilkörper einen Schließstift und einen den Schließstift umgebenden Druckbeaufschlagungskragen aufweisen. Die Druckbeaufschlagungsfläche ist vorzugsweise vergleichsweise groß, um mit geringem Druck eine Verlagerung des Schließstiftes zu bewirken.

Insbesondere von Vorteil ist es, wenn der Ventilkörper in einem Außenbereich und/oder im Bereich dieser Druckbeaufschlagungsfläche in sich verformbar ausgebildet ist und sich unter der Wirkung des Flüssigkeitsdrucks elastisch verformt. Der Außenbereich ist bei einersolchen Gestaltungfestgelegt, insbesondere am Innenbauteil, beispielsweise durch umlaufende Fixierung in einer Nut des Innenbauteils.

Der Speicherbelüftungskanal kann ein bei angenommener Kappe stets offener Verbindungskanal zwischen einer äußeren Umgebung und dem Innenraum des Flüssigkeitsspeichers sein, wobei vorzugsweise mindestens ein Filter vorgesehen ist, der die einströmende Luft von Verunreinigungen reinigt, bevor diese in Kontakt mit der Flüssigkeit gelangt. Bei einer aufwändigeren Bauart kann ein Ventil vorgesehen sein, welches nur bei Unterdruck im Flüssigkeitsspeicher öffnet.

Zum Zwecke des Austrags von Flüssigkeit wird Flüssigkeit stromaufwärts des Auslassventils druckbeaufschlagt, so dass das Auslassventil geöffnet und Flüssigkeit ausgetragen wird. Die Erfindung ist dabei nicht auf eine bestimmte Technik der Druckbeaufschlagung beschränkt. Eine besonders bevorzugte Ausgestaltung sieht jedoch vor, dass der Flüssigkeitsspender als Quetschflaschenspender ausgebildet ist. Er weist in einem solchen Falle einen manuell elastisch verformbaren Flaschenkörper auf, der zum Zwecke der Druckbeaufschlagung der Flüssigkeit zusammengedrückt wird.

Vorzugsweise weist der Flüssigkeitsspeicher ein Innenvolumen von weniger als 250 ml, insbesondere 100 ml, vorzugsweise weniger als 50 ml, auf.

### KURZBESCHREIBUNG DER ZEICHNUNGEN

Weitere Vorteile und Aspekte der Erfindung ergeben sich aus den Ansprüchen und aus der nachfolgenden Beschreibung eines bevorzugten Ausführungsbeispiels der Erfindung, das nachfolgend anhand der Figuren erläutert ist.
Fig. 1 zeigt einen erfindungsgemäßen Flüssigkeitsspender in Gesamtdarstellung ohne aufgesetzte Kappe.
Fig. 2 zeigt den Austragkopf des Flüssigkeitsspenders in geschnittener perspektivischer Darstellung.
Fig. 3 zeigt den Austragkopf in ungeschnittener perspektivischer Darstellung.
Fig. 5 und 6 zeigen zwei Schnittebenen des Austragkopfes, deren jeweilige Schnittebene anhand von Fig. 4 verdeutlicht ist.

### DETAILLIERTE BESCHREIBUNG DER AUSFÜHRUNGSBEISPIELE

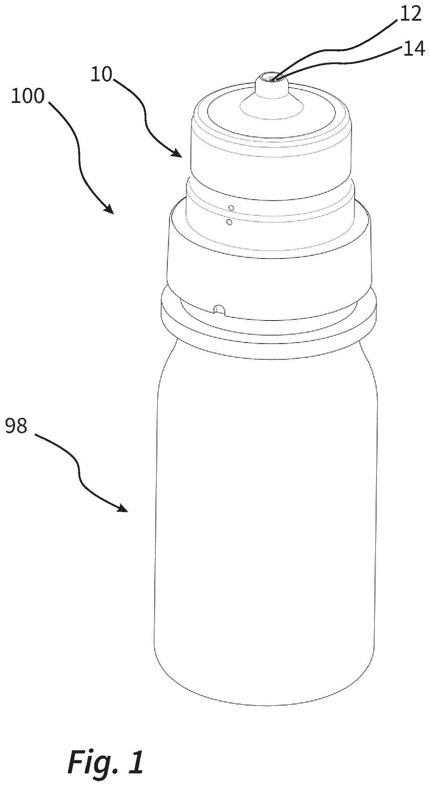

Fig. 1 zeigt einen exemplarischen erfindungsgemäßen Flüssigkeitsspender 100 in einer Gesamtdarstellung, jedoch ohne Schutzkappe 120.

Der Flüssigkeitsspender 100 verfügt über einen Flaschenkörper 98 aus mit geringer Kraft elastisch verformbarem Kunststoff, eine so genannte Quetschflasche (Squeeze Bottle). Am Flaschenhals des Flaschenkörpers 98 ist ein Austragkopf 10 befestigt, vorliegend mittels einer Schnappverbindung.

Der Austragkopf 10 weist eine Austragöffnung 12 auf. Da der exemplarische Flüssigkeitsspender als Tropfenspender ausgebildet ist, ist im Bereich der Austragöffnung ein Tropfenbildungsmittel vorgesehen, vorliegend in Form einer die Austragöffnung 12 umgebenden Tropfenbildungsfläche 14, die durch eine umlaufende Abrisskante begrenzt wird.

Der Spender wird bestimmungsgemäß in einer Überkopflage verwendet, in der die Austragöffnung 12 nach unten weist. In dieser Stellung wird der Flaschenkörper 98 manuell zusammengedrückt. Dies bewirkt, dass unter dem Eindruck des erhöhten Flüssigkeitsdrucks im Flüssigkeitsspeicher ein Auslassventil 20 im Austragkopf 10 öffnet und Flüssigkeit dosiert zur Tropfenbildungsfläche 14 gelangen lässt. Dort sammelt sich die Flüssigkeit, bis das gewünschte Tropfenvolumen erreicht ist und der Tropfen sich von der Tropfenbildungsfläche löst.

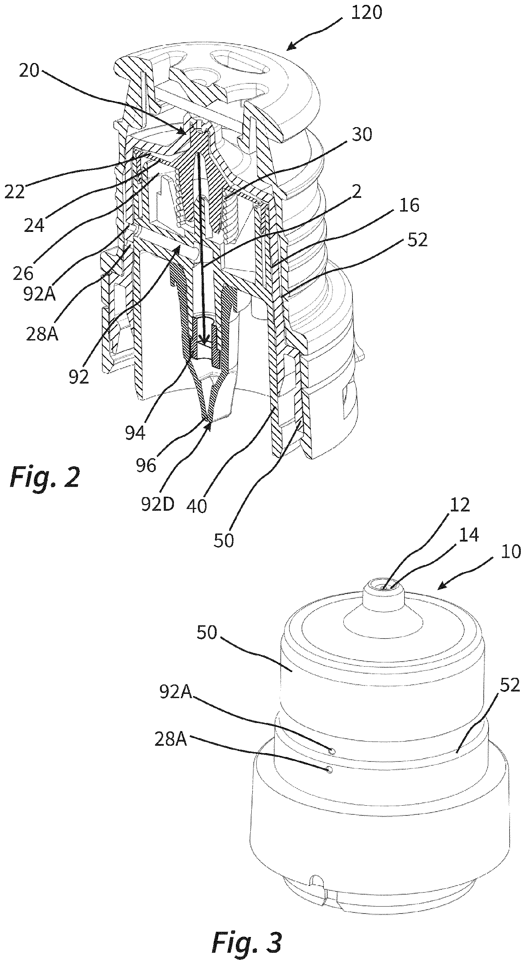

Fig. 2 zeigt den Austragkopf 10 in einer geschnittenen perspektivischen Darstellung mit aufgesetzter Schutzkappe 120. Die Hauptbauteile des Austragkopfes 10 sind ein Innenbauteil 40 und ein Außenbauteil 50, das in einer Montagerichtung 2 auf das Innenbauteil aufgeschoben ist.

Zwischen den beiden Bauteilen 40, 50 ist ein Ventilkörper 24 vorgesehen, der aus elastischem Kunststoff gefertigt ist. Dieser Ventilkörper weist zentral einen Schließstift 24A auf, welcher sich im geschlossenen Zustand des Auslassventils an eine die Austragöffnung 12 umgebende Innenwandung des Außenbauteils 50 anlegt. Der Schließstift 24A geht in einen Druckbeaufschlagungskragen 24B über, der den Ventilstift scheibenförmig umgibt. An dessen äußerem Rand ist ein Befestigungssteg 24C vorgesehen, der umlaufend in eine Befestigungsnut des Innenbauteils 40 eingeschoben ist.

Im Bereich des Druckbeaufschlagungskragens 24B ist zwischen dem Ventilkörper 24 und dem Außenbauteil 50 eine Druckkammer22 gebildet. Diese wird durch einen Austragkanal 16 mit Flüssigkeit gespeist, der sich von einem nicht dargestellten Einlass auf Seite des Flüssigkeitsspeichers 90 über einen Ringraum zwischen dem Innenbauteil 40 und dem Außenbauteil 50 bis in die Druckkammer 22 erstreckt. Auf der der Druckkammer 22 abgewandten Seite des Ventilkörpers 24 ist eine Ventilkammer 26 vorgesehen, in der eine Rückstellfeder 30 vorgesehen ist, die den Ventilkörper 24 in Richtung seiner Schließstellung kraftbeaufschlagt.

Wird der Flüssigkeitsdruck im Flüssigkeitsspeicher 90 erhöht, so erhöht sich auch der Druck in der Druckkammer 22 und der Ventilkörper 24 wird bei wird unter Verformung im Bereich des Druckbeaufschlagungskragens 24B nach unten verlagert. Das Volumen der Ventilkammer 26 wird dabei verringert.

Sowohl der Flüssigkeitsspeicher 90 als auch die Ventilkammer 26 sind zumindest phasenweise mit einer umgebenden Atmosphäre verbunden, um einen Druckausgleich zu schaffen. Im Falle des Flüssigkeitsspeichers 90 dient der hierfür vorgesehene Speicherbelüftungskanal 92 primär dem Zweck, das Nachströmen von Luft zu gestatten, wenn zuvor Flüssigkeit ausgetragen wurde. Im Falle der Ventilkammer 26 dient der Ventilbelüftungskanal 28 dem Zweck, zu verhindern, dass über einen längeren Zeitraum in der Ventilkammer 26 ein vom Umgebungsdruck abweichender Druck besteht, da dies das Öffnungsverhalten des Auslassventils 20 ändern könnte.

Erfindungsgemäß ist vorgesehen, dass die beiden Belüftungskanäle 28, 92 voneinander getrennt sind. Abseits des Austragkanals 16 besteht demnach keine weitere durch die Bauweise des Austragkopfes begründete Wirkkopplung zwischen den jeweiligen Drücken in den Belüftungskanälen 28, 92. Herrscht beispielsweise ein Unterdruck im Flüssigkeitsspeicher 90, da zuvor Flüssigkeit ausgetragen worden ist, so bewirkt dieser keinen Unterdruck in der Ventilkammer 26.

Die beiden Belüftungskanäle 28, 92 weisen jeweils Einlässe 28A, 92A an der Außenseite des Außenbauteils 50 auf. Dies wird anhand von Fig. 3 verdeutlicht. Bei dieser Gestaltung sind die beiden Einlässe 28A, 92A auf der gleichen des Austragkopfes 10 vorgesehen, jedoch in Montagerichtung 2 gegeneinander versetzt. Diese Anordnung auf der gleichen Seite gestattet die Nutzung eines vergleichsweise einfachen Werkzeugs, da die Einlässe 28A, 92A durch einen gemeinsamen Schieber im Werkzeug erzeugt werden können. Es sind jedoch auch anderweitige Gestaltung möglich und fallweise sinnvoll, insbesondere eine Anordnung der Einlässe 28A, 92A auf gegenüberliegenden Seiten. Zwischen den Einlässen 28A, 92A, ist eine Stufe 52 vorgesehen, an der sich der Außendurchmesser des Außenbauteils 50 verringert. Diese Stufe ist im Zusammenwirken mit der Schutzkappe 120 von Vorteil. Wie anhand von Fig. 2 ersichtlich ist, liegt die Innenseite der Schutzkappe 120 im aufgesetzten Zustand umlaufend an der Stufe 52 an, so dass sie die beiden Einlässe 28A, 92A voneinander isoliert sind.

Diese Isolation durch die Schutzkappe 120 bewirkt, dass auch bei aufgesetzter Schutzkappe 120 der Flüssigkeitsspeicher 90 und die Ventilkammer 26 gegenüber isoliert sind. Wird also die Kappe nach Verwendung und damit bewirktem Unterdruck im Flüssigkeitsspeicher 90 sofort aufgesetzt, bevor es zum Druckausgleich im Flüssigkeitsspeicher 90 kommt, so kann der im Flüssigkeitsspeicher 90 und im Belüftungskanals 92 herrschende Unterdruck keinen Einfluss auf die Verhaltensweise des Auslassventils nehmen. Ein ungewolltes Auslaufen des Spenders, beispielsweise im Reisegepäck, wird dadurch verhindert.

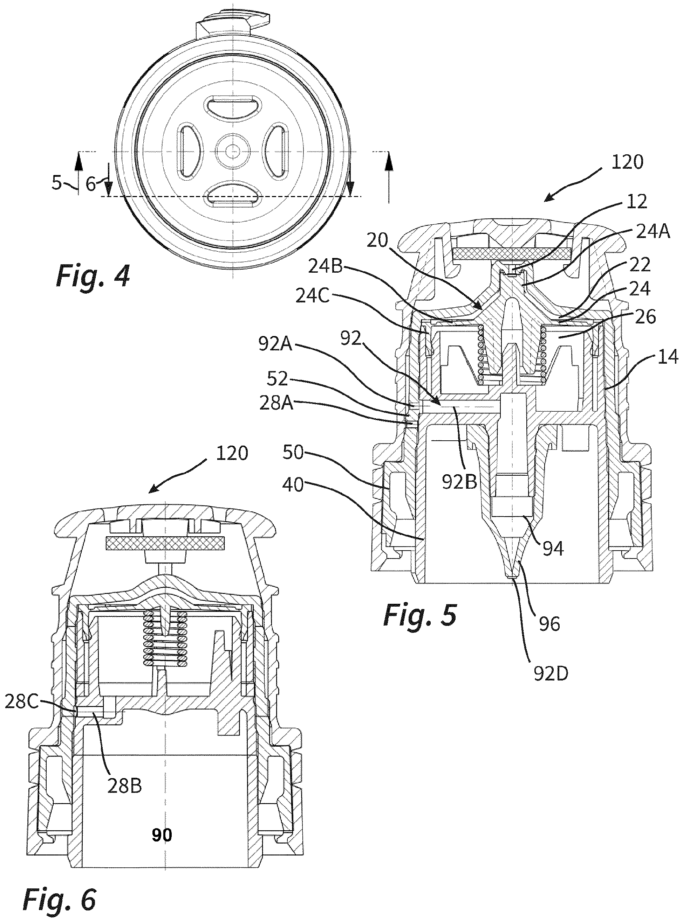

Anhand der Fig. 4 bis 6 wird der Verlauf der Belüftungskanäle 28, 92 verdeutlicht. Fig. 4 zeigt den Austragkopf 10 des erfindungsgemäßen Spenders 100 von oben. Durch die Bezugszeichen 5 und 6 werden zwei Schnittebenen gekennzeichnet, deren Schnittdarstellungen in den Fig. 5 und 6 dargestellt sind.

Die Schnittebene 5 verläuft durch eine Mittelachse des Spenders und seine zentrische Austragöffnung. In dieser Ebene verläuft der radiale Kanalteilabschnitt 92B des Speicherbelüftungskanals 92. Die Schnittebene 6 ist dazu parallel nach außen verschoben. In dieser Ebene verläuft ein radialer Kanalteilabschnitt 28b des Ventilbelüftungskanals 28.

Bezugnehmend auf Fig. 6 wird zunächst der Verlauf des Ventilbelüftungskanals 28 näher erläutert. Der Ventilbelüftungskanal 28 weist ausgehend von der Ventilkammer 26 einen kurzen Teilabschnitt auf, der parallel zur Montagerichtung 2 verläuft. Hieran schließt sich der genannte geradlinige Kanalteilabschnitt 28B an, der eine Außenseite des Innenbauteils 40 durchstößt. Dabei mündet dieser Kanalteilabschnitt 28B aufgrund seiner exzentrischen und nicht exakt radialen Anordnung nicht senkrecht auf die Außenseite. Ein sich an den Kanalteilabschnitt 28B anschließender Kanalteilabschnitt 28C wird durch einen ringabschnittsförmigen Kanalteilabschnitt 28C gebildet, der sich zwischen dem Innenbauteil 40 und dem Außenbauteil 50 umlaufend erstreckt, vorliegend etwa um 30° umlaufend, bis zum im Außenbauteil 50 vorgesehenen Einlass 28A.

Der in Fig. 5 gut erkennbare Speicherbelüftungskanal 92 erstreckt sich vom Einlass 92A, der das Au-ßenbauteil 50 durchstößt, in einen radialen Kanalteilabschnitt 92B, der bei der hier dargestellten Relativdrehstellung von Außenbauteil 50 und Innenbauteil40 mit dem Einlass 92A fluchtet. Das Au-ßenbauteil50 und das Innenbauteil40 könnten jedoch auch in anderer Relativdrehstellungverbunden sein, so dass sich zwischen dem Einlass 92A und dem Kanalteilabschnitt 92B im Innenbauteil 40, wie bereits zum Ventilbelüftungskanal beschrieben, ein ringabschnittsförmiger Kanalteilabschnitt zwischen dem Innenbauteil 40 und dem Außenbauteil 50 ergibt.

Innenseitig schließt sich an den radialen Kanalteilabschnitt 92B ein axialer Kanalteilabschnitt an, der partiell durch einen Ventilkörper 96 gebildet ist, welcher den Auslass 92D des Speicherbelüftungskanal 92 bildet. Weiterhin kann hier eine Filtereinheit 94 vorgesehen sein, um die einströmende Luft von Verunreinigungen zu befreien.

Wie anhand der parallelen Schnittebenen 5, 6 und der darin angeordneten Belüftungskanäle 92, 28 ersichtlich ist, sind die Belüftungskanäle zueinander parallel angeordnet, jedoch bezogen auf die in Fig. 4 dargestellte Ebene gegeneinander versetzt. Dies gestattet es, das Innenbauteil 40 mit nur einem Schieber zur Bildung der Kanalteilabschnitte 92B, 28B ausstatten zu müssen und dennoch diese voneinander zu beabstanden, so dass die jeweiligen Kanäle im Innenbauteil ausreichenden Bauraum aufweisen.

## Patentansprüche

1. Tropfenspender (100) mit den folgenden Merkmalen:
a. der Tropfenspender (100) verfügt über einen Flüssigkeitsspeicher (90), und
b. der Tropfenspender (100) verfügt über einen am Flüssigkeitsspeicher (90) befestigten Austragkopf (10) mit einer Austragöffnung (12), die über einen Austragkanal (16) mit dem Flüssigkeitsspeicher (90) verbunden ist, und
c. der Austragkopf (10) weist im Austragkanal (16) ein Auslassventil (20) mit einem an eine Druckkammer (22) angrenzenden Ventilkörper (24) auf, welcher durch Flüssigkeitsdruck in der Druckkammer (22) zum Zwecke des Öffnens der Austragöffnung (12) zumindest partiell verlagerbar ist, und
d. der Ventilkörper (24) weist einen Schließstift (24A), welcher sich in einem geschlossenen Zustand des Auslassventils (20) an eine die Austragöffnung (12) umgebende Innenwandung anlegt, und einen den Schließstift (24A) umgebenden Druckbeaufschlagungskragen (24B) auf, und
e. das Auslassventil (20) weist eine Ventilkammer (26) auf, die bezogen auf den Ventilkörper (24) gegenüberliegend zur Druckkammer (22) angeordnet ist, und
f. zum Zwecke des Druckausgleichs im Flüssigkeitsspeicher (90) ist ein Speicherbelüftungskanal (92) vorgesehen, und
g. zum Zwecke des Druckausgleichs zwischen der Ventilkammer (26) und einer äußeren Umgebung ist ein Ventilbelüftungskanal (28) vorgesehen,
**gekennzeichnet durch** die folgenden weiteren Merkmale:
h. der Speicherbelüftungskanal (92) und der Ventilbelüftungskanal (28) weisen voneinander getrennte Einlässe (92A, 28A) an einer Außenseite des Austragkopfes (10) auf und
i. Tropfenbildungsmittel (14) sind im Bereich der Austragöffnung (12 vorgesehen.

2. Tropfenspender (100) nach Anspruch 1 mit den folgenden weiteren Merkmalen:
a. der Austragkopf (10) weist ein Innenbauteil (40) und ein Außenbauteil (50) auf, wobei das Außenbauteil (50) auf das Innenbauteil (40) in einer Montagerichtung aufgeschoben ist, und
b. der Speicherbelüftungskanal (92) und der Ventilbelüftungskanal (28) erstrecken sich jeweils durch das Innenbauteil (40) und das Außenbauteil (50).

3. Tropfenspender (100) nach Anspruch 2 mit dem folgenden weiteren Merkmal:
a. der Speicherbelüftungskanal (92) und/oder der Ventilbelüftungskanal (28) weisen einen Kanalteilabschnitt (28C) auf, der als ringabschnittsförmiger Kanalteilabschnitt (28C) zwischen dem Außenbauteil (50) und dem Innenbauteil (40) gebildet ist.

4. Tropfenspender (100) nach Anspruch 1 oder 2 mit dem folgenden weiteren Merkmal:
a. die Einlässe (92A, 28A) sind an einer seitlichen Außenseite des Austragkopfes (10) vorgesehen, insbesondere am Außenbauteil (50), wobei die Einlässe (92A, 28A) bezogen auf die Montagerichtung gegeneinander versetzt sind und wobei vorzugsweise zwischen den Einlässen (92A, 28A) eine umlaufende Stufe (52) an einer Außenseite des Austragkopfes (10) vorgesehen ist.

5. Tropfenspender (100) nach einem der vorstehenden Ansprüche mit dem folgenden weiteren Merkmal:
a. der Speicherbelüftungskanal (92) und der Ventilbelüftungskanal weisen jeweils geradlinige Kanalteilabschnitte (92B, 28B) auf, die ein Innenbauteil (40) des Tropfenspenders (100) durchdringen, die bezogen auf eine Ebene, deren Normalenvektor mit einer Austragrichtung übereinstimmt, gegeneinander angewinkelt oder insbesondere parallel und beabstandet zueinander angeordnet sind.

6. Tropfenspender (100) nach einem der vorstehenden Ansprüche mit den folgenden weiteren Merkmalen:
a. der Austragkopf (10) weist eine abnehmbare Schutzkappe (120) auf, die im aufgesetzten Zustand die Austragöffnung schützt, und
b. die Schutzkappe (120) isoliert im aufgesetzten Zustand mindestens einen der Einlässe (92A, 28A) gegenüber einer umgebenden Atmosphäre.

7. Tropfenspender (100) nach Anspruch 6 mit dem folgenden weiteren Merkmal:
a. die Schutzkappe (120) isoliert im aufgesetzten Zustand beide Einlässe (92A, 28A) voneinander und jeweils gegenüber einer umgebenden Atmosphäre.

8. Tropfenspender (100) nach einem der vorstehenden Ansprüche mit dem folgenden weiteren Merkmal:
a. die Tropfenbildungsmittel (14) umfassen eine die Austragöffnung (12) außenseitig umgebende Tropfenbildungsfläche.

9. Tropfenspender (100) nach einem der vorstehenden Ansprüche mit dem folgenden weiteren Merkmal:
a. der Speicherbelüftungskanal (92) weist zwischen einem außenseitigen Einlass (92A) und einem speicherseitigen Auslass (92D) mindestens einen Filter (94) und/oder ein Einlassventil auf.

10. Tropfenspender (100) nach einem der vorstehenden Ansprüche mit dem folgenden weiteren Merkmal:
a. der Tropfenspender (100) ist als Quetschflaschenspender ausgebildet und weist einen zum Zwecke der Druckbeaufschlagung manuell elastisch verformbaren Flaschenkörper (98) auf, der den Flüssigkeitsspeicher (90) umgibt.

11. Tropfenspender (100) nach einem der vorstehenden Ansprüche mit dem folgenden weiteren Merkmal:
a. in der Ventilkammer (26) ist eine Rückstellfeder (30) für den Ventilkörper (24) angeordnet.

## Claims

1. Drop dispenser (100) with the following features:
a. the drop dispenser (100) has a liquid reservoir (90), and
b. the drop dispenser (100) has a discharge head (10) which is fastened to the liquid reservoir (90) and has a discharge opening (12) which is connected via a discharge channel (16) to the liquid reservoir (90), and,
c. in the discharge channel (16), the discharge head (10) has an outlet valve (20) with a valve body (24) which adjoins a pressure chamber (22) and can be moved at least partially by way of liquid pressure in the pressure chamber (22) for the purpose of opening the discharge opening (12), and
d. the valve body (24) has a closing pin (24A), which, in a closed state of the outlet valve (20), bears against an inner wall which surrounds the discharge opening (12), and a pressurizing collar (24B) which surrounds the closing pin (24A), and
e. the outlet valve (20) has a valve chamber (26) which, in relation to the valve body (24), is arranged so as to lie opposite the pressure chamber (22), and
f. a reservoir ventilating channel (92) is provided for the purpose of pressure equalization in the liquid reservoir (90), and
g. a valve ventilating channel (28) is provided for the purpose of pressure equalization between the valve chamber (26) and an outer surrounding area,
**characterized by** the following further features:
h. the reservoir ventilating channel (92) and the valve ventilating channel (28) have inlets (92A, 28A) which are separate from one another on an outer side of the discharge head (10), and
i. drop formation means (14) are provided in the region of the discharge opening (12).

2. Drop dispenser (100) according to Claim 1, having the following further features:
a. the discharge head (10) has an inner component (40) and an outer component (50), wherein the outer component (50) is pushed onto the inner component (40) in a mounting direction, and
b. the reservoir ventilating channel (92) and the valve ventilating channel (28) extend in each case through the inner component (40) and the outer component (50).

3. Drop dispenser (100) according to Claim 2, having the following further feature:
a. the reservoir ventilating channel (92) and/or the valve ventilating channel (28) have/has a channel part portion (28C) which is formed as an annular section-shaped channel part portion (28C) between the outer component (50) and the inner component (40).

4. Drop dispenser (100) according to Claim 1 or 2, having the following further feature:
a. the inlets (92A, 28A) are provided on a lateral outer side of the discharge head (10), in particular on the outer component (50), wherein the inlets (92A, 28A) are offset with respect to one another in relation to the mounting direction, and wherein a circumferential step (52) is preferably provided between the inlets (92A, 28A) on an outer side of the discharge head (10).

5. Drop dispenser (100) according to one of the preceding claims, having the following further feature:
a. the reservoir ventilating channel (92) and the valve ventilating channel each have rectilinear channel part portions (92B, 28B) which penetrate an inner component (40) of the drop dispenser (100), are angled away with respect to one another in relation to a plane, the perpendicular vector of which coincides with a discharge direction, or are arranged, in particular, parallel to and spaced apart from one another.

6. Drop dispenser (100) according to one of the preceding claims, having the following further features:
a. the discharge head (10) has a removable protective cap (120) which protects the discharge opening in the fitted state, and
b. the protective cap (120) isolates at least one of the inlets (92A, 28A) with respect to a surrounding atmosphere in the fitted state.

7. Drop dispenser (100) according to Claim 6, having the following further feature:
a. in the fitted state, the protective cap (120) isolates the two inlets (92A, 28A) from one another and in each case with respect to a surrounding atmosphere.

8. Drop dispenser (100) according to one of the preceding claims, having the following further feature:
a. the drop formation means (14) comprise a drop formation surface which surrounds the discharge opening (12) on the outer side.

9. Drop dispenser (100) according to one of the preceding claims, having the following further feature:
a. the reservoir ventilating channel (92) has at least one filter (94) and/or one inlet valve between an outer-side inlet (92A) and a reservoir-side outlet (92D).

10. Drop dispenser (100) according to one of the preceding claims, having the following further feature:
a. the drop dispenser (100) is configured as a squeeze bottle dispenser and has a bottle body (98) which can be deformed manually elastically for the purpose of pressurization and surrounds the liquid reservoir (90).

11. Drop dispenser (100) according to one of the preceding claims, having the following further feature:
a. a restoring spring (30) for the valve body (24) is arranged in the valve chamber (26).

## Revendications

1. Distributeur de gouttes (100), avec les particularités suivantes :
a) le distributeur de gouttes (100) dispose d'un réservoir de liquide (90), et
b) le distributeur de gouttes (100) dispose d'une tête de distribution (10) fixée au réservoir de liquide (90) avec un orifice de distribution (12) qui est relié au réservoir de liquide (90) par un canal de distribution (16), et
c) la tête de distribution (10) présente dans le canal de distribution (16) une vanne de sortie (20) pourvue d'un corps de vanne (24) adjacent à une chambre de pression (22) et qui peut être déplacé au moins partiellement par la pression de liquide dans la chambre de pression (22) afin d'ouvrir l'ouverture de distribution (12), et
d) le corps de vanne (24) présente une goupille de fermeture (24A) qui est adjacente dans un état fermé de la vanne de sortie (20) à une paroi intérieure entourant l'ouverture de distribution (12) et une collerette de mise sous pression (24B) entourant la goupille de fermeture (24A), et
e) la vanne de sortie (20) présente une chambre de vanne (26) qui est disposée à l'opposé de la chambre de pression (22), par rapport au corps de vanne (24), et
f) un canal de ventilation de réservoir (92) est prévu à des fins d'équilibrage de pression dans le réservoir de liquide (90), et
g) un canal de ventilation de vanne (28) est prévu à des fins d'équilibrage de pression entre la chambre de vanne (26) et un environnement extérieur,
**caractérisé par** les particularités supplémentaires suivantes :
h) le canal de ventilation de réservoir (92) et le canal de ventilation de vanne (28) présentent des entrées (92A, 28A) séparées l'une de l'autre sur une face extérieure de la tête de distribution (10), et
i) des moyens de formation de gouttes (14) sont prévus au niveau de l'ouverture de sortie (12).

2. Distributeur de gouttes (100) selon la revendication 1, avec les caractéristiques supplémentaires suivantes :
a) la tête de distribution (10) présente un composant intérieur (40) et un composant extérieur (50), le composant extérieur (50) étant emmanché sur le composant intérieur (40) dans une direction de montage, et
b) le canal de ventilation de réservoir (92) et le canal de ventilation de vanne (28) s'étendent respectivement à travers le composant intérieur (40) et le composant extérieur (50).

3. Distributeur de gouttes (100) selon la revendication 2, avec la caractéristique supplémentaire suivante :
a) le canal de ventilation de réservoir (92) et/ou le canal de ventilation de vanne (28) présentent une section de canal (28C) qui est formée comme une section de canal (28C) en forme de partie annulaire entre le composant extérieur (50) et le composant intérieur (40).

4. Distributeur de gouttes (100) selon la revendication 1 ou 2, avec la caractéristique supplémentaire suivante :
a) les entrées (92A, 28A) sont prévues sur une face extérieure latérale de la tête de distribution (10), en particulier sur le composant extérieur (50), dans lequel les entrées (92A, 28A) sont décalées l'une par rapport à l'autre par rapport à la direction de montage, et dans lequel de préférence un gradin périphérique (52) est prévu entre les entrées (92A, 28A), sur une face extérieure de la tête de distribution (10).

5. Distributeur de gouttes (100) selon l'une quelconque des revendications précédentes, avec la caractéristique supplémentaire suivante :
a) le canal de ventilation de réservoir (92) et le canal de ventilation de vanne présentent respectivement des sections de canal rectilignes (92B, 28B) qui pénètrent dans un composant intérieur (40) du distributeur de gouttes (100), qui sont coudées l'une par rapport à l'autre ou sont disposées en particulier en parallèle et de manière espacée l'une de l'autre, par rapport à un plan dont le vecteur normal coïncide avec une direction de distribution.

6. Distributeur de gouttes (100) selon l'une quelconque des revendications précédentes, avec les caractéristiques supplémentaires suivantes :
a) la tête de distribution (10) présente un capuchon de protection amovible (120) qui protège à l'état emboîté l'ouverture de distribution, et
b) le capuchon de protection (120) isole à l'état emboîté au moins l'une des entrées (92A, 28A) par rapport à une atmosphère ambiante.

7. Distributeur de gouttes (100) selon la revendication 6, avec la caractéristique supplémentaire suivante :
a) le capuchon de protection (120) isole à l'état emboîté les deux entrées (92A, 28A) l'une par rapport à l'autre et respectivement par rapport à une atmosphère ambiante.

8. Distributeur de gouttes (100) selon l'une quelconque des revendications précédentes, avec la caractéristique supplémentaire suivante :
a) les moyens de formation de gouttes (14) comprennent une surface de formation de gouttes entourant côté extérieur l'ouverture de distribution (12).

9. Distributeur de gouttes (100) selon l'une quelconque des revendications précédentes, avec la caractéristique supplémentaire suivante :
a) le canal de ventilation de réservoir (92) présente entre une entrée côté extérieur (92A) et une sortie côté réservoir (92D) au moins un filtre (94) et/ou une vanne d'entrée.

10. Distributeur de gouttes (100) selon l'une quelconque des revendications précédentes, avec la caractéristique supplémentaire suivante :
a) le distributeur de gouttes (100) est réalisé sous forme de flacon souple et présente un corps de flacon (98) à déformation élastique manuelle à des fins de mise sous pression qui entoure le réservoir de liquide (90).

11. Distributeur de gouttes (100) selon l'une quelconque des revendications précédentes, avec la caractéristique supplémentaire suivante :
a) un ressort de rappel (30) pour le corps de vanne (24) est disposé dans la chambre de vanne (26).
